# EUROPEAN PATENT APPLICATION

(11) **EP 2 390 342 A1**
(43) Date of publication of application: **30.11.2011**
(21) Application number: 10733579.6
(22) Date of filing: 22.01.2010
(51) Int. Cl.: C12P 7/56, B01D 61/44, B01J 39/04

(54) **METHOD FOR PRODUCING D-LACTIC ACID, AND METHOD FOR INCREASING OPTICAL PURITY OF D-LACTIC ACID OR YIELD OF D-LACTIC ACID RELATIVE TO SUGAR IN LACTIC ACID**

(30) Priority: 23.01.2009 JP 2009013234
(71) Applicant: Agribioindustry Inc., Sapporo-shi, Hokkaido 063-0062 (JP)
(72) Inventor: MATSUSHIMA Tokuo, Sapporo-shi Hokkaido 063-0062 (JP); KANAMARU Kiyotaka, Sapporo-shi Hokkaido 060-0042 (JP)
(74) Representative: Prüfer & Partner GbR European Patent Attorneys
(86) International application number: PCT/JP2010/050852
(87) International publication number: WO 2010/084972

(57) **Abstract**

Disclosed is a method for producing D-lactic acid by allowing a microorganism to utilize a purified treacle and culturing the microorganism. Also disclosed is a method for increasing the optical purity of D-lactic acid in lactic acid. Specifically disclosed is a method for producing D-lactic acid by allowing a microorganism to utilize a treacle and culturing the microorganism, which involves a step of purifying the treacle by means of an ion exchange technique. The method enables the efficient production of lactic acid which contains D-lactic acid having an optical purity of 99.40 % e.e. or more.

## Description

### Technical Field

The present invention relates to a method for producing D-lactic acid and a method for increasing the optical purity of D-lactic acid in lactic acid or the yield of D-lactic acid in lactic acid from sugar.

### Background Art

Recently, development of bioresource (biomass)-derived biodegradable polymers has been actively carried out because of depletion of and an increase in the price of fossil resources, the environmental issues, etc. From a carbon-neutral perspective, polylactic acid, which is a representative biodegradable polymer, is drawing attention as a sustainable polymer. As polylactic acid, not only poly-L-lactic acid (PLLA) and poly-D-lactic acid (PDLA) but also a random polymer of L-lactic acid and D-lactic acid, a block polymer of L-lactic acid and D-lactic acid, or a stereocomplex polymer composed of a mixture of PLLA and PDLA has been synthesized, and the development of polylactic acid is in progress.

Two kinds of optical isomers, namely L-lactic acid and D-lactic acid, exist for a lactic acid monomer, which is a raw material of the aforementioned polylactic acid. Particularly, utilization of D-lactic acid as a raw material of polymers and other compounds requiring the skeleton of D-lactic acid is anticipated in the fields of medicine and agricultural chemical, and a high optical purity of D-lactic acid in lactic acid and a high yield of D-lactic acid in lactic acid from sugar are demanded.

Examples of a conventional method of efficient production of D-lactic acid can include a method including screening for naturally existing microorganisms that efficiently produce D-lactic acid and allowing the microorganisms to perform fermentation under appropriate conditions and a method of allowing transformants produced by introducing a gene encoding D-lactate dehydrogenase into microorganisms to perform fermentation under appropriate conditions.

For instance, as an example of the former method, Patent Literature 1 discloses a method of allowing microorganisms producing lactic acid containing D-lactic acid of an optical purity of 90% e.e. or more and belonging to the genus Bacillus to perform fermentation under certain conditions. Also, as examples of the latter method, Patent Literature 2 discloses a method for producing D-lactic acid using transformants produced by introducing a gene encoding D-lactate dehydrogenase derived from Lactobacillus plantarum into high pyruvic acid-producing yeast, Patent Literature 3 discloses a method for producing D-lactic acid under aerated conditions using transformants produced by introducing a gene encoding D-lactate dehydrogenase derived from Lactobacillus plantarum and the like into E. coli, and Patent Literature 4 discloses a method for producing D-lactic acid using transformants produced by introducing a gene encoding D-lactate dehydrogenase derived from Bacillus laevolacticus into yeast. Further, Patent Literature 5 discloses a method for producing D-lactic acid by continuous fermentation using the aforementioned high D-lactic acid-producing microorganisms.

Meanwhile, treacle, which is generated in the production of unrefined sugar from cane juice, purification of unrefined sugar, or production of beet sugar from beet, contains 40 to 60% sugar; therefore, it has been conventionally used as the carbon source for microbial fermentation. For example, Non Patent Literature 1 discloses that beet treacle from which excess potassium ions, formic acid, and acetic acid are removed by cation exchange process, ether extraction process, and electrodialysis can be preferably used as an itaconic acid fermentation medium.

### Citation List

### Patent Literature

Patent Literature 1: Japanese Patent Laid-Open No.2003-88392
Patent Literature 2: Japanese Patent Laid-Open No.2002-136293
Patent Literature 3: Japanese Patent Laid-Open No.2005-102625
Patent Literature 4: Japanese Patent Laid-Open No.2007-74939
Patent Literature 5: Japanese Patent Laid-Open No.2008-104451

### Non Patent Literature

Non Patent Literature 1: Mitsutoshi Nakagawa et al., Research Bulletin of Obihiro University I, 17, 7 to 12 (1990)

### Summary of Invention

### Technical Problem

However, there is absolutely no disclosure in Patent Literatures 1 to 5 regarding purification of treacle using the ion exchange resin method to allow microorganisms to utilize the treacle thus purified, and further, Non Patent Literature 1 does not disclose at all the production of D-lactic acid and a method for increasing the optical purity of D-lactic acid in lactic acid or the yield of D-lactic acid in lactic acid from sugar.

An object of the present invention is to provide a method for efficiently producing D-lactic acid by allowing microorganisms to utilize purified treacle and culturing the microorganisms, i.e., a method for producing lactic acid containing D-lactic acid of high optical purity and a method for increasing the optical purity of D-lactic acid in lactic acid or the yield of D-lactic acid in lactic acid from sugar.

### Solution to Problem

The present inventors conducted intensive research. As a result, the present inventors have found that lactic acid containing D-lactic acid with an optical purity of 99.40% e.e. or more can be produced by purifying treacle using the ion exchange resin method and allowing microorganisms to utilize the treacle thus purified, and the yield of L-lactic acid from sugar can be suppressed while the yield of D-lactic acid from sugar can be increased by 64.1% or more by allowing microorganisms to utilize treacle having one or two or more ions selected from the group consisting of potassium ions, magnesium ions, phosphate ions, hydrogen phosphate ions, dihydrogen phosphate ions, and sulfate ions added thereto, thereby completing each of the following inventions.

(1) A method for producing D-lactic acid by allowing microorganisms to utilize treacle and culturing the microorganisms, the method comprising the step of purifying the treacle using an ion exchange resin method.

(2) The method for producing D-lactic acid according to (1), wherein the microorganisms are lactic acid bacteria.

(3) The method for producing D-lactic acid according to (1) or (2), wherein the ion exchange resin method uses two or more ion exchange resins and comprises the step of contacting treacle having been contacted with a cation exchange resin or an anion exchange resin with an anion exchange resin.

(4) The method for producing D-lactic acid according to any of (1) to (3), wherein the ion exchange resin method is a column method using two or more ion exchange resin columns and comprising allowing treacle having flowed through a cation exchange resin column or an anion exchange resin column to flow through an anion exchange resin column.

(5) The method for producing D-lactic acid according to (4), wherein, in the column method, an amount of treacle extruded from the anion exchange resin column used first is 1.8 to 4 parts by volume per part by volume of the anion exchange resin.

(6) The method for producing D-lactic acid according to any of (1) to (5), wherein the ion exchange resin method or the column method uses a heated ion exchange resin or ion exchange resin column.

(7) The method for producing D-lactic acid according to any of (1) to (6), wherein the treacle has one or two or more ions selected from the group consisting of potassium ions, magnesium ions, phosphate ions, hydrogen phosphate ions, dihydrogen phosphate ions, and sulfate ions added thereto.

(8) A method for increasing an optical purity of D-lactic acid in lactic acid, comprising the step of purifying treacle using an ion exchange resin method.

(9) The method for increasing an optical purity of D-lactic acid in lactic acid according to (8), wherein the microorganisms are lactic acid bacteria.

(10) The method for increasing an optical purity of D-lactic acid in lactic acid according to (8) or (9), wherein the ion exchange resin method uses two or more ion exchange resins and comprises the step of contacting treacle having been contacted with a cation exchange resin or an anion exchange resin with an anion exchange resin.

(11) The method for increasing an optical purity of D-lactic acid in lactic acid according to any of (8) to (10), wherein the ion exchange resin method is a column method using two or more ion exchange resin columns and comprising allowing treacle having flowed through a cation exchange resin column or an anion exchange resin column to flow through an anion exchange resin column.

(12) The method for increasing an optical purity of D-lactic acid in lactic acid according to (11), wherein, in the column method, an amount of treacle extruded from the anion exchange resin column used first is 1.8 to 4 parts by volume per part by volume of anion exchange resin.

(13) The method for increasing an optical purity of D-lactic acid in lactic acid according to any of (8) to (12), wherein the ion exchange resin method or the column method uses a heated ion exchange resin or ion exchange resin column.

(14) A method for increasing a yield of D-lactic acid from sugar, comprising the step of allowing microorganisms to utilize treacle having one or two or more ions selected from the group consisting of potassium ions, magnesium ions, phosphate ions, hydrogen phosphate ions, dihydrogen phosphate ions, and sulfate ions added thereto and culturing the microorganisms.

(15) Lactic acid comprising D-lactic acid, an optical purity of the D-lactic acid or a yield of the D-lactic acid from sugar being increased using the method according to any of (8) to (14).

### Advantageous Effects of Invention

Comparing to the existing methods including allowing microorganisms to utilize treacle and culturing the microorganisms, the present invention enables more efficient production of lactic acid containing D-lactic acid of high optical purity, and further, can increase the yield of D-lactic acid from sugar; therefore, D-lactic acid can be economically provided.

### Brief Description of Drawings

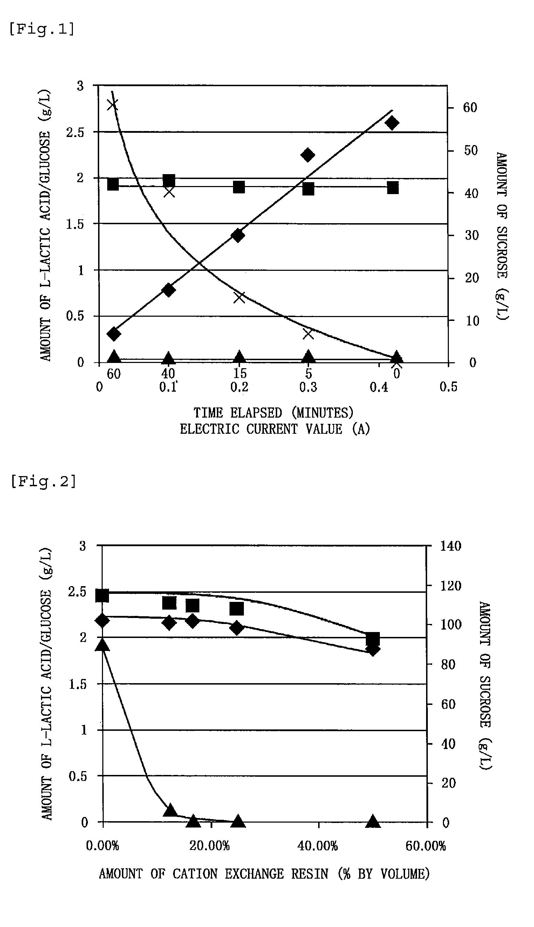

[Figure 1] Figure 1 is a graph showing a relationship of the contents of glucose, sucrose, and L-lactic acid in electrodialysis-purified HB treacle obtained by purifying HB treacle by electrodialysis relative to the time elapsed and the electric current value. In the graph, × represents the time elapsed (minute) and the electric current value (A), and ▲, ■, and ◆ each represent the amount of glucose (g/L), the amount of sucrose (g/L), and the amount of L-lactic acid (g/L).
[Figure 2] Figure 2 is a graph showing a relationship of the contents of glucose, sucrose, and L-lactic acid in cation exchange-purified HB treacle obtained by purifying HB treacle by a cation exchange resin relative to the amount of cation exchange resin. In the graph, ▲, ■, and ◆ each represent the amount of glucose (g/L), the amount of sucrose (g/L), and the amount of L-lactic acid (g/L).

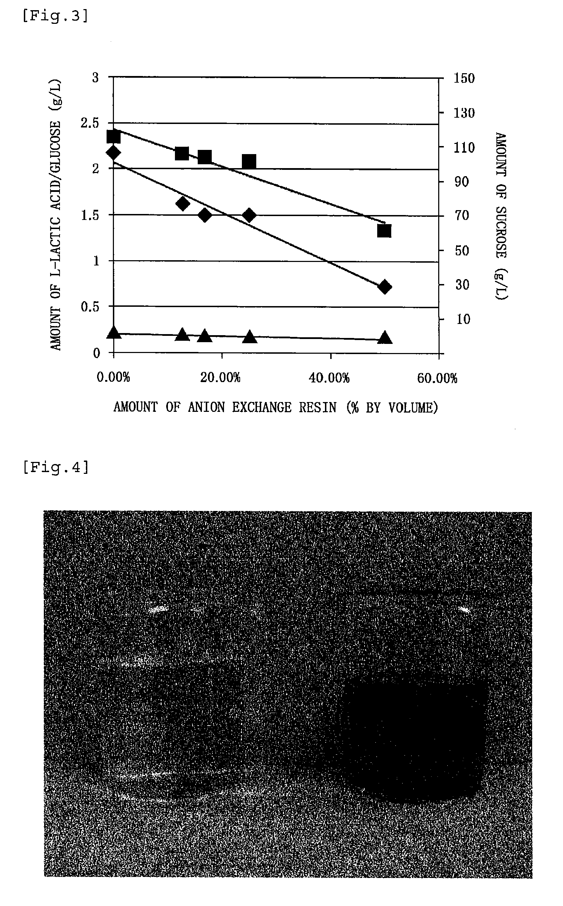

[Figure 3] Figure 3 is a graph showing a relationship of the contents of glucose, sucrose, and L-lactic acid in anion exchange-purified HB treacle obtained by purifying HB treacle by an anion exchange resin relative to the amount of anion exchange resin. In the graph, ▲, ■, and ◆ each represent the amount of glucose (g/L), the amount of sucrose (g/L), and the amount of L-lactic acid (g/L).
[Figure 4] Figure 4 is a diagram (photograph) showing the decoloring effect of the ion exchange resin method. A sample on the observer's right represents HB treacle and a sample on the observer's left represents the cation and anion exchange-purified HB treacle.

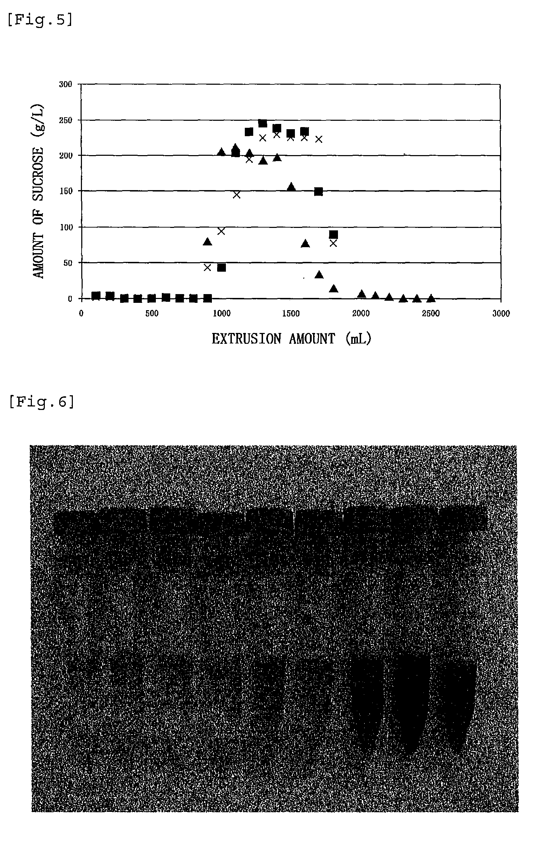

[Figure 5] Figure 5 is a graph showing a relationship between the amount of treacle extruded by the ion exchange resin method and the sucrose content in the anion exchange-purified HB treacle. In the graph, ■, ×, and ▲ represent Sample Nos. 1, 2, and 3, respectively.
[Figure 6] Figure 6 is a diagram (photograph) showing the decoloring effect of the ion exchange resin method observed in 1000 to 1800 mL of extruded HB treacle.

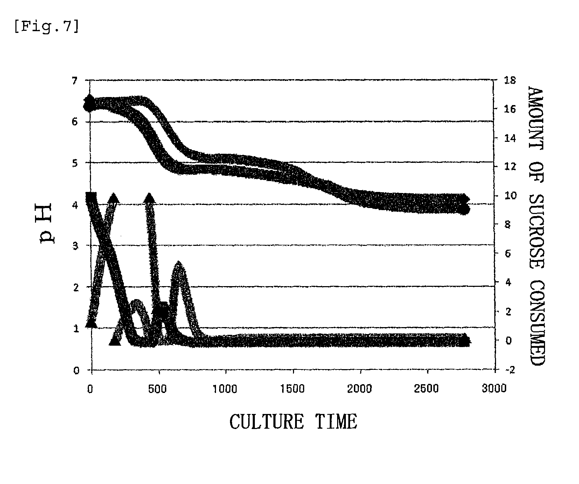

[Figure 7] Figure 7 is a graph showing a relationship among the culture time before and after scale-up, the pH value, and the amount of sucrose consumed. In the graph, ◆ and • represent shifts in the pH value in a 10 L jar fermenter and a 90 L jar fermenter, respectively, and ▲ and ■ represent the amounts of sucrose consumed in a 10 L jar fermenter and a 90 L jar fermenter, respectively.

### Description of Embodiments

Hereinbelow, the method for producing D-lactic acid and the method for increasing the optical purity of D-lactic acid in lactic acid or the yield of D-lactic acid in lactic acid from sugar according to the present invention will be described in detail. The method for producing D-lactic acid according to the present invention is a method for producing D-lactic acid by allowing microorganisms to utilize treacle and culturing the microorganisms.

Treacle used in the present invention includes not only a liquid containing sugar (treacle in the narrow sense) but also by-products of the production of sugar (molasses) and treacle that could fall under both treacle in the narrow sense and molasses. Examples of such treacle can include cane treacle, beet treacle, refinder's molasses, hydrol, various types of fermentation waste liquors, and residual sugars. Examples of the cane treacle can include high-test molasses, which is cane sugar that is reduced to amorphous sugar, and examples of the beet treacle include beet thick juice, which is concentrated in advance before the production step of beet sugar is started, HA treacle, which is discharged from the process in which ion exchange resin treatment is incorporated into the purification step of beet sugar, and HB treacle, which is discharged from the process in which the Steffen process is introduced into the purification step of beet sugar. Also, the treacle used in the present invention may be treacle prepared as a medium and treacle to which water, water-soluble solvents, and the like are appropriately added, and also, one appropriately containing a substance that can be selected within a range such that the feature of the present invention is not impaired, for example, sugars such as glucose and sucrose, minerals such as sodium, calcium, iron, manganese, zinc, copper, magnesium, and potassium, D-lactic acid, and L-lactic acid.

Here, "D-lactic acid" as referred to in the present invention is intended to encompass not only lactic acid containing D-lactic acid having 100% optical purity but also "lactic acid containing D-lactic acid of high optical purity", in which the optical purity of D-lactic acid is less than 100%. Herein, "D-lactic acid" and "lactic acid containing D-lactic acid of high optical purity" may be interchangeably used. That is, in the present invention, "the method for producing D-lactic acid" and "the method for producing lactic acid containing D-lactic acid of high optical purity" are interchangeably used.

The "lactic acid containing D-lactic acid of high optical purity" refers to, irrespective of the concentration of lactic acid in a fermentation liquor obtainable by allowing microorganisms to utilize treacle and culturing the microorganisms, lactic acid containing D-lactic acid having an optical purity of at least 90% e.e. or more, preferably lactic acid containing D-lactic acid having an optical purity of 95% e.e. or more, more preferably lactic acid containing D-lactic acid having an optical purity of 96% e.e. or more, further preferably lactic acid containing D-lactic acid having an optical purity of 97% e.e. or more, further more preferably lactic acid containing D-lactic acid having an optical purity of 98% e.e. or more, and most preferably lactic acid containing D-lactic acid having an optical purity of 99% e.e. or more.

The "microorganism" used in the present invention includes, in addition to eubacteria and archaea capable of producing D-lactic acid, eukaryotes such as algae, protists, fungi, and slime molds capable of producing D-lactic acid, very small animals such as annelids, and transformants that have acquired a D-lactic acid-producing ability, and preferable microorganisms can include lactic acid bacteria.

Examples of the lactic acid bacteria used in the present invention can include lactic acid bacteria belonging to the genus Bacillus such as Bacillus coagulans, lactic acid bacteria belonging to the genus Lactobacillus such as Lactobacillus delbrueckii, Lactobacillus acidophilus, Lactobacillus casei, Lactobacillus fructivorans, Lactobacillus hilgardii, Lactobacillus paracasei, Lactobacillus rhamnosus, Lactobacillus plantarum, Lactobacillus bulgaricus, Lactobacillus acidophilus, and Lactobacillus brevis, lactic acid bacteria belonging to the genus Bifidobacterium such as Bifidobacterium bifidum, Bifidobacterium adolescentis, Bifidobacterium longum, and Bifidobacterium lactis, lactic acid bacteria belonging to the genus Enterococcus such as Enterococcus faecalis, Enterococcus faecium, Enterococcus caseliflavus, and Enterococcus sulfreus, lactic acid bacteria belonging to the genus Lactococcus such as Lactococcus lactis, Lactococcus cremoris, Lactococcus diacetylactis, Lactococcus plantarum, and Lactococcus rafinolactis, lactic acid bacteria belonging to the genus Pediococcus such as Pediococcus damnosus and Pediococcus pentosaceus, lactic acid bacteria belonging to the genus Leuconostoc such as Leuconostoc mesenteroides and Leuconostoc citreum, lactic acid bacteria belonging to the genus Carnobacterium such as Carnobacterium divergens and Carnobacterium piscicola, and lactic acid bacteria belonging to the genus Streptococcus such as Streptococcus thermophilus, Streptococcus faecalis, and Streptococcus pyogenes.

It is to be noted that, in the present invention, not only commercial, known microorganisms that are deposited in a public institution and the like or registered in a public database but also confidential or newly-isolated microorganisms may be used. Further, two or more kinds of microorganisms may be used.

Any medium can be appropriately selected and used as the medium for allowing microorganisms to utilize treacle and culturing the microorganisms according to the present invention as long as it contains treacle and is capable of culturing the aforementioned microorganisms. For example, whether it is a natural medium or synthetic medium, or whether it is a liquid medium or solid medium is not questioned. The aforementioned medium contains a carbon source, a nitrogen source, inorganic salts, and the like. As the carbon source, in addition to sugars such as glucose and sucrose contained in the treacle, for example, sugars such as glucose, sucrose, fructose, maltose, galactose, and lactose, alcohols such as methanol, ethanol propanol, and glycerol, and organic acids such as citric acid, malic acid, succinic acid, maleic acid, and fumaric acid can be added, etc. to be used. Examples of the nitrogen source that can be used include, in addition to natural nitrogen sources such as peptone, yeast extract, malt extract, meat extract, casamino acids, corn steep liquor, soy protein, defatted soybean, and cottonseed meal, organic nitrogen sources such as urea, and inorganic nitrogen sources such as sodium nitrate, ammonium nitrate, and ammonium sulfate. Examples of the inorganic salts that can be used include salts of potassium, sodium, magnesium, iron, manganese, cobalt, and zinc, sulfate salts, and phosphate salts, and specific examples include potassium chloride, sodium chloride, magnesium sulfate, ferrous sulfate, manganese sulfate, cobalt chloride, zinc sulfate, potassium dihydrogen phosphate, potassium phosphate, and sodium phosphate. Although potassium dihydrogen phosphate (KH₂PO₄) and magnesium sulfate (MgSO₄) are preferably used, they are also preferably dissolved and used in the form of potassium ions, magnesium ions, phosphate ions, hydrogen phosphate ions, potassium dihydrogen phosphate ions, and sulfate ions. Besides those noted above, specific amino acids, vitamins, and the like can be used as needed.

No particular limitation is imposed on a sterilization method of a medium in the present invention, and sterilization can be performed by any method that can be selected by those skilled in the art. For example, a medium can be autoclaved at 121°C for 15 minutes. Also, when there is a possible risk that the components of the medium undergo chemical reactions during autoclave sterilization and cause compositional changes, each of the components can be separately autoclaved.

In the culture in the present invention, conditions can be appropriately selected or modified depending on the microorganisms selected to be cultured and the situations of production and purification of D-lactic acid. For example, when lactic acid bacteria belonging to the genus Lactobacillus are used, culturing is preferably performed under anaerobic conditions. It is to be noted that, in order to perform culturing under anaerobic conditions, the culture can be placed statically, or anaerobic culturing can also be performed while passing inert gas. As the inert gas, carbon dioxide, nitrogen, ammonia, argon, and the like can be used, and the amount of gas flow, means of gas supply, etc. can be appropriately selected according to the situations of production and purification of D-lactic acid. Also, shaking culture or spinner culture can be performed, and as the form of culture, batch culture, semi-batch culture, continuous culture, and the like can be employed. Further, in order to suppress foaming during culturing, an antifoaming agent can be appropriately selected and added to the medium. The amount of the antifoaming agent added is not particularly limited either, and the amount normally used by those skilled in the art can be employed.

The culture temperature is desirably 20°C to 50°C, preferably 35°C to 47°C. The culture time is normally 24 hours to 120 hours. Because pH of the culture liquid decreases during culturing along with the production and accumulation of D-lactic acid, pH is desirably maintained at 2.0 to 10.0 using an alkali solution, ammonia, calcium carbonate, calcium hydroxide, and the like. However, pH is maintained preferably at 3.0 to 8, and more preferably at 6.0 to 6.5, and this pH adjustment can be achieved using an alkali solution, ammonia, calcium carbonate, calcium hydroxide, and the like.

A method for collecting D-lactic acid from culture products such as a culture liquid obtained by culturing can be performed by any method that can be selected by those skilled in the art. Examples of the method include a method of directly distilling a culture product after acidifying it, a method of distilling after formation of lactide by D-lactic acid, a method of distilling after esterification of D-lactic acid by addition of alcohols and catalysts, a method of extracting D-lactic acid in an organic solvent, a method of separating D-lactic acid by an ion exchange column, a method of concentrating and separating D-lactic acid by electrodialysis, and a method of a combination of the above methods; however, a method including subjecting the aforementioned culture product from which bacterial bodies are removed to protein removal and salt substitution, filtering and concentrating the resulting product to give crystals, appropriately recrystallizing the crystals, and separating the resulting crystals by an acid is preferable.

Although no particular limitation is imposed on a method for determining the optical purity of D-lactic acid contained in the lactic acid thus obtained, for example, it can be determined by calculating according to the following formula:
Optical purity (%) = 100 × (D - L) / (D + L): wherein, L represents the concentration of L-lactic acid and D represents the concentration of D-lactic acid. Besides this, examples of the above method include a method using High Performance Liquid Chromatography (HPLC) and a method using F-kit (Roche Diagnostics K.K., Nippon Boehringer Ingelheim Co., Ltd., etc.).

Also, in the method for producing D-lactic acid according to the present invention, the aforementioned treacle is purified by electrodialysis and/or the ion exchange resin method.

Electrodialysis is a membrane separation process utilizing an ion exchange membrane and electricity, and is generally performed with an electrodialysis apparatus equipped with a cation exchange membrane and an anion exchange membrane. No limitation is imposed on the ion exchange membrane, the electrodialysis apparatus, and the like used in the electrodialysis in the present invention, and they can be appropriately selected and used.

The ion exchange resin method is a purification method for replacement of ion species (ion exchange), wherein a resin takes up ions contained in the contacting electrolyte solution in exchange for releasing other ion species held by the resin. In the ion exchange resin method in the present invention, besides an anion exchange resin, a cation exchange resin can be further used, and irrespective of whether they are strong base or weak base or whether they are strong acid or weak acid, respectively, commercial products, etc. can be appropriately selected and used. Also, the aforementioned ion exchange resin is not limited to a popular product having a parent body composed of copolymers of styrene and divinylbenzene but includes ion exchange membranes used for diffusion dialysis, etc.

To the ion exchange resin method in the present invention, either a batch method in which an ion exchange resin is thrown into treacle, followed by stirring, or a column method in which a column is filled with an ion exchange resin, through which treacle is passed in an upflow or downflow direction can be applied. Examples of a column method in which treacle is passed through in a downflow direction can include the elution development such as ion exchange chromatography and the leakage method.

Here, "contact" as used in the present invention refers to such a degree of contact that permits the aforementioned replacement of ion species (ion exchange) to take place on a part of or the entire resin, and may be interchangeably used with, for example, "immersing", "passing a solution through", and "flowing through." Also, "purification" refers to removal, reduction, or inhibition of the production of at least any of L-lactic acid, glucose, ions, heavy metals, and the like contained in treacle by the aforementioned contact. An embodiment in which L-lactic acid is removed or reduced, or the production thereof is inhibited is preferable, an embodiment in which L-lactic acid is removed or reduced, or the production thereof is inhibited, while at least any of glucose, cations, and heavy metals is removed or reduced is more preferable, and an embodiment in which L-lactic acid is removed or reduced, or the production thereof is inhibited, while at least any of glucose, cations, and heavy metals is removed or reduced and decolorization is performed, is further preferable.

Further, the ion exchange resin method in the present invention may be an ion exchange resin method including the step of contacting treacle having been contacted with an anion exchange resin with an anion exchange resin again, or an ion exchange resin method including the step of contacting treacle having been contacted with a cation exchange resin with an anion exchange resin. That is, the ion exchange resin method in the present invention may be an ion exchange resin method including the step of contacting treacle having been contacted with a cation or anion exchange resin with an anion exchange resin immediately or after storage, etc., or may be an ion exchange resin method including, before or after the above step, the step of contacting the treacle with one or two or more ion exchange resins.

Also, in the method for producing D-lactic acid according to the present invention, the ion exchange resin method may be a column method using two or more ion exchange resin columns, wherein the column method includes allowing treacle having flowed through a cation exchange resin column or an anion exchange resin column to flow through an anion exchange resin column.

Further, the column method in the present invention is preferably a column method wherein the amount of treacle extruded from the anion exchange resin column used first is 1.8 to 4 parts by volume per part by volume of anion exchange resin.

Herein, the cation exchange resin column and the anion exchange resin column in the present invention include not only an ion exchange resin column in which the column is filled with only a cation exchange resin or an anion exchange resin but also a cation exchange resin column and an anion exchange resin column formed in such a way that the column is filled with a cation exchange resin and an anion exchange resin so that each of them functions as a cation exchange resin column or an anion exchange resin column, for example, an ion exchange resin column filled with a cation exchange resin and an anion exchange resin in such a way that both are mixed and an ion exchange resin column filled with a cation exchange resin and an anion exchange resin in such a way that these resins form a multilayer.

Also, the ion exchange resin (the cation exchange resin and/or the anion exchange resin) and/or the ion exchange resin column (the cation exchange resin column and/or the anion exchange resin column) used in the present invention is preferably heated. In this case, the aforementioned ion exchange resin and/or ion exchange resin column is heated preferably up to less than 90°C, more preferably up to 88°C or below, further preferably up to 86°C or below, further more preferably up to 84°C or below, and most preferably up to 82°C or below.

Also, the amount of treacle extruded, namely, the amount of treacle having been passed through the column is preferably 1.8 to 4 parts by volume, more preferably 2 to 3.2 parts by volume per part by volume of the anion exchange resin used first.

Hereinbelow, the method for producing D-lactic acid and the method for increasing the optical purity of D-lactic acid in lactic acid or the yield of D-lactic acid in lactic acid from sugar according to the present invention will be described based on Examples. It should be noted that the technical scope of the present invention is not limited to the features demonstrated by these Examples.

### Examples

### <Example 1> Study using HB treacle

### (1) Measurement of HB treacle

In HB treacle, the contents of glucose and sucrose were measured by multifunctional biosensor BF-5 (Oji Scientific Instruments Co., Ltd.); the contents of D-lactic acid and L-lactic acid were measured by a D/L-lactic acid measurement kit (SL11-0011; Oji Scientific Instruments Co., Ltd.) and a D-lactic acid measurement kit (SL11-0018; Oji Scientific Instruments Co., Ltd.); the contents of sodium, calcium, iron, manganese, zinc, copper, and magnesium were measured by Inductively Coupled Plasma Atomic Emission Spectrometry (ICP-AES) or Inductively Coupled Plasma Optical Emission Spectrometry (ICP-OES) (ICPE-9000; Shimadzu Corporation); and potassium was measured by atomic absorption spectrophotometer (AA-6300; Shimadzu Corporation). As a result, HB treacle did not contain D-lactic acid, while the contents of glucose, sucrose, and L-lactic acid were 1.9 wt% (g/100 g), 52.6 wt% (g/100 g), and 2.03 g/L, respectively.

### (2) Measurement of electrodialysis-purified HB treacle

Based on the results of the measurement of the sucrose content in the present Example 1 (1), an aqueous solution of HB treacle was prepared by adding distilled water to HB treacle at a ratio of 160 g of HB treacle to 1 L of distilled water so that the total sucrose content was 100 g/L. Then, HB treacle was purified by electrodialysis to give electrodialysis-purified HB treacle. The electrodialysis was performed in an electrodialysis tank filled with the total amount of the aqueous solution of HB treacle, wherein the electrodialysis tank was formed by setting the 3.5 cm-long, 9.5 cm-wide, and 1.0 mm-thick cation and anion exchange membranes (both are AC 220-10; ASTOM Corporation) on a compact electrodialysis apparatus (ASTOM Corporation).

A relationship between the contents of glucose, sucrose, and L-lactic acid in the electrodialysis-purified HB treacle and the time elapsed and the electric current value are shown in Figure 1. As shown in Figure 1, it can be confirmed that while the L-lactic acid content in the electrodialysis-purified HB treacle tends to decrease in proportion to the value of electric current being conducted, the contents of glucose and sucrose in the electrodialysis-purified HB treacle tend to be kept almost constant irrespective of the time elapsed and the electric current value. Further, the contents of glucose, sucrose, D-lactic acid, L-lactic acid, sodium, calcium, iron, manganese, zinc, copper, magnesium, and potassium in the electrodialysis-purified HB treacle thus obtained were measured by the same technique as that employed in Example 1 (1). It should be noted that the decoloring effect of electrodialysis was not confirmed in the electrodialysis-purified HB treacle thus obtained.

### (3) Purification of HB treacle by the ion exchange resin method

After preparing an aqueous solution of HB treacle by the same technique as that employed in the present Example 1 (2), it was purified by the ion exchange resin method to give cation exchange-purified HB treacle and anion exchange-purified HB treacle. For the ion exchange, 1 m-high two glass columns having a diameter of 5 cm were produced, and the ion exchange was performed by allowing the total amount of the aqueous solution of HB treacle to flow through each of the following columns; the columns filled with neither a cation (strong acid ion) exchange resin nor an anion (strong base ion) exchange resin (both were Amberlite; Organo Corporation) and the columns filled with each of the cation exchange resin and the anion exchange resin up to 20 v/v%, 40 v/v%, and 60 v/v% of the column volume.

A relationship between the amount of the cation exchange resin and the contents of glucose, sucrose, and L-lactic acid in the cation exchange-purified HB treacle is shown in Figure 2, and a relationship between the amount of the anion exchange resin and the contents of glucose, sucrose, and L-lactic acid in the anion exchange-purified HB treacle is shown in Figure 3. From Figure 2, it can be confirmed that while the contents of sucrose and L-lactic acid in the cation exchange-purified HB treacle tends to gradually decrease along with an increase in the amount of the cation exchange resin, the glucose content in the cation exchange-purified HB treacle tends to sharply decrease, irrespective of the amount of the cation exchange resin. Also, from Figure 3, it can be confirmed that while the contents of sucrose and L-lactic acid in the anion exchange-purified HB treacle tend to decrease in proportion to an increase in the amount of the anion exchange resin, the glucose content in the anion exchange-purified HB treacle tends to slightly decrease according to the amount of the anion exchange resin.

That is, from Figures 2 and 3, it is found that, when purifying HB treacle by the ion exchange resin method, the contents of glucose and L-lactic acid can be efficiently reduced by allowing HB treacle having flowed through a cation exchange resin column where it contacted a cation exchange resin to flow through an anion exchange resin column to allow it to contact an anion exchange resin or allowing HB treacle having flowed through an anion exchange resin column where it contacted an anion exchange resin to flow through an anion exchange resin column to allow it to contact an anion exchange resin.

### (4) Purification of HB treacle by performing anion exchange after cation exchange

By the same technique as that employed in the present Example 1 (2), 7 L of an aqueous solution of HB treacle was prepared. Then, firstly, the total amount of aqueous solution of HB treacle thus prepared was allowed to flow through a cation exchange resin column filled with a cation (strong acid ion) exchange resin (Amberlite; Organo Corporation) up to 60 v/v% of the column volume, and collected. Subsequently, the total amount of the solution thus collected was allowed to flow through an anion exchange resin column filled with an anion (strong base ion) exchange resin (Amberlite; Organo Corporation) up to 60 v/v% of the column volume, and collected, whereby the HB treacle was purified. The contents of glucose, sucrose, D-lactic acid, L-lactic acid, sodium, calcium, iron, manganese, zinc, copper, magnesium, and potassium in the cation and anion exchange-purified HB treacle thus obtained were measured by the same technique as that employed in the present Example 1 (1). Further, the decoloring effect of the ion exchange resin method was confirmed in the cation and anion exchange-purified HB treacle thus obtained. This is shown in Figure 4 (photograph).

### (5) Fermentation using the electrodialysis-purified HB treacle and the cation and anion exchange-purified HB treacle and measurement of the resulting lactic acid

Seven liters of each of the electrodialysis-purified HB treacle and the cation and anion exchange-purified HB treacle were prepared, to which 5 g/L of yeast extract was added as a nitrogen source, followed by adjustment of pH to 6.0 to 6.5. The HB treacles thus prepared were autoclaved at 121°C for 15 minutes and then cooled, to which 30 g/L of calcium carbonate was added to prepare media. Each medium was prepared in an original amount of 70% of a 10 L jar fermenter (7.0 L), into which 2% (v/v) of Lactobacillus delbrueckii strains, which are known lactic acid bacteria isolated from the sugar production step, were inoculated, followed by 48 hours of shaking culture at 45°C and 150 rpm/minute. After culturing, each fermentation liquid was collected and subjected to centrifugation at 8000 rpm for 10 minutes to remove the bacterial bodies. To the resulting supernatants, calcium hydroxide (slaked lime) was added so that pH was 10.0 or higher, followed by heating at 80°C for one hour to carry out protein removal and salt substitution. The resulting solutions were cooled to room temperature, followed by filtration. The filtrates were concentrated and the crystals precipitated were filtered out, which were washed with a small amount of distilled water and then recrystallized. The filtrates separated by filtration were concentrated again, and the crystals precipitated were filtered out and then recrystallized. This step was repeated several times to give white crystals of calcium lactate. To this, distilled water was added and the crystals were dissolved at 60°C. After cooling, concentrated sulfuric acid was added so that pH was approximately 1.5 to 1.7 to separate a supernatant containing lactic acid from calcium sulfate. The supernatant obtained by filtration was concentrated under reduced pressure at 65°C to remove remaining calcium sulfate, whereby respective lactic acids (i.e., the electrodialysis-purified HB treacle lactic acid and the cation and anion exchange-purified HB treacle lactic acid) were obtained.

The optical purities of D-lactic acid in the electrodialysis-purified HB treacle lactic acid and the cation and anion exchange-purified HB treacle lactic acid were determined by calculating according to the following formula:
Optical purity (%) = 100 × (D - L) / (D + L): wherein, L represents the concentration of L-lactic acid and D represents the concentration of D-lactic acid.
Subsequently, the contents of glucose, sucrose, D-lactic acid, L-lactic acid, sodium, calcium, iron, manganese, zinc, copper, magnesium, and potassium in the electrodialysis-purified HB treacle lactic acid and the cation and anion exchange-purified HB treacle lactic acid were measured by the same technique as that employed in the present Example 1 (1).

### <Comparative Example 1> Fermentation using HB treacle and measurement of the resulting lactic acid

Fermentation was performed using unpurified HB treacle and the resulting lactic acid was measured. By the same technique as that employed in Example 1 (2), 7 L of an aqueous solution of HB treacle (a total sucrose amount of 100 g/L) was prepared. Subsequently, by the same technique as that employed in Example 1 (5), media were prepared and fermentation was carried out, followed by concentration to give lactic acid (i.e., HB treacle lactic acid). The optical purity of D-lactic acid and the contents of glucose, sucrose, D-lactic acid, L-lactic acid, sodium, calcium, iron, manganese, zinc, copper, magnesium, and potassium in the HB treacle lactic acid thus obtained were measured by the same technique as that employed in Example 1 (1) and (5). The results of the measurement of Example 1 and the present Comparative Example 1 are shown in Table 1.

**[Table 1]**

| Item | Unit | HB treacle | Electrodialysi anion s-purified HB treacle | Cation and exchange-purified HB treacle | Eleotrodialysi s-purified HB treacle lactic acid | Cation and anion exchange-purified HB treacle lactic acid | HB treacle lactic acid |
|---|---|---|---|---|---|---|---|
| Glucose | g/100g | 1,9 | 0.219 | 0.226 | ND | 19.66 | ND |
| Sucrose | g/100g | 52.6 | 94.9 | 90 | 0 | 0 | 0 |
| D-lactic acid | g/L | 0 | 0 | 0 | 94.9 | 94.1 | 91.6 |
| L-lactic acid | g/L | 2.03 | 0.219 | 0 | 2.26 | 0.329 | 2.03 |
| Optical purity of D-lactic acid | % | - | - | - | 95.35 | 99.30 | 95.66 |
| Sodium | mg/L | 4230 | 510 | 0 | NT | 0 | - |
| Potassium | mg/L | 9480 | 763 | ND | NT | ND | - |
| Calcium | mg/L | 44.8 | 18.5 | 18.3 | NT | 17.3 | - |
| Iron | mg/L | 3.013 | 1.043 | 0.03 | NT | 0 | - |
| Manganese | mg/L | 2.291 | 0.588 | 0 | NT | 0 | - |
| Zinc | mg/L | 5.689 | 2.479 | 0 | NT | 0 | - |
| Copper | mg/L | 0.151 | 0 | 0 | NT | 0 | - |
| Magnesium | mg/L | 6.582 | 0.972 | 0 | NT | 0.02 | - |
| Presence or absence of decolorization | - | - | Absent | Present | Absent | Present | - |

From Table 1, it can be confirmed that the contents of glucose, L-lactic acid, sodium, potassium, calcium, iron, manganese, zinc, copper, and magnesium in the electrodialysis-purified HB treacle and the cation and anion exchange-purified HB treacle are drastically reduced compared to the contents of these substances in HB treacle. Thus, it can be confirmed that the ratios of the sucrose contents in the electrodialysis-purified HB treacle and the cation and anion exchange-purified HB treacle are increased. It can be also confirmed that the cation and anion exchange-purified HB treacle lactic acid exhibits an extremely higher value of optical purity of D-lactic acid than does the HB treacle lactic acid, and also, potassium, calcium, and magnesium remain in the cation and anion exchange-purified HB treacle. Among those, calcium is considered attributable to calcium hydroxide added for salt substitution. Further, although no decolorization can be confirmed in the electrodialysis-purified HB treacle lactic acid obtained in a similar manner to the electrodialysis-purified HB treacle, decolorization can be confirmed in the cation and anion exchange-purified HB treacle lactic acid, similarly to the cation and anion exchange-purified HB treacle.

### <Example 2> Study using beet thick juice

### (1) Measurement of beet thick juice

The contents of glucose, sucrose, D-lactic acid, L-lactic acid, sodium, calcium, iron, manganese, zinc, copper, magnesium, and potassium in beet thick juice were measured by the same technique as that employed in Example 1 (1). As a result, the beet thick juice did not contain glucose, D-lactic acid, and L-lactic acid, and the sucrose content was 61.2 wt% (g/100 g).

### (2) Purification of beet thick juice by electrodialysis

Based on the result of the measurement of the sucrose content in the present Example 2 (1), 7 L of an aqueous solution of beet thick juice was prepared by adding distilled water to beet thick juice at a ratio of 160 g of beet thick juice to 1 L of distilled water so that the total sucrose amount was 100 g/L. Then, in a similar manner to Example 1 (2), the resulting aqueous solution of beet thick juice was subjected to electrodialysis to give electrodialysis-purified beet thick juice.

### (3) Purification of the beet thick juice by the ion exchange resin method

By the same technique as that employed in the present Example 2 (2), 7 L of an aqueous solution of beet thick juice was prepared. Then, firstly, the total amount of the aqueous solution of beet thick juice was allowed to pass through the cation exchange resin column used in Example 1 (4), and collected. Subsequently, the total amount of the aqueous solution of beet thick juice thus obtained was allowed to pass through the anion exchange resin column used in Example 1 (4) to give cation and anion exchange-purified beet thick juice.

### (4) Fermentation using the electrodialysis-purified beet thick juice and the cation and anion exchange-purified beet thick juice and measurement of the resulting purified lactic acid

By the same technique as that employed in Example 1 (5), media were prepared from the electrodialysis-purified beet thick juice and the cation and anion exchange-purified beet thick juice and fermentation was carried out, followed by concentration to give respective lactic acids (i.e., electrodialysis-purified beet thick juice lactic acid and cation and anion exchange-purified beet thick juice lactic acid). The optical purities of D-lactic acid and the contents of glucose, sucrose, D-lactic acid, L-lactic acid, sodium, calcium, iron, manganese, zinc, copper, magnesium, and potassium in the electrodialysis-purified beet thick juice lactic acid and the cation and anion exchange-purified beet thick juice lactic acid thus obtained were measured by the same technique as that employed in Example 1 (1) and (5).

### <Comparative Example 2> Fermentation using the beet thick juice and measurement of the resulting lactic acid

Fermentation was performed using unpurified beet thick juice and the resulting lactic acid was measured. By the same technique as that employed in Example 2 (2), 7 L of an aqueous solution of beet thick juice (a total sucrose amount of 120 g/L) was prepared. Subsequently, by the same technique as,that employed in Example 1 (5), media were prepared and fermentation was carried out, followed by concentration to give lactic acid (i.e., beet thick juice lactic acid). The optical purity of D-lactic acid and the contents of glucose, sucrose, D-lactic acid, L-lactic acid, sodium, calcium, iron, manganese, zinc, copper, magnesium, and potassium in the beet thick juice lactic acid thus obtained were measured by the same technique as that employed in Example 1 (1) and (5). The results of the measurement of Example 2 and the present Comparative Example 2 are shown in Table 2.

**[Table 2]**

| Item | Unit | Beat | Electrodialysis-purified beet thick juice lactic acid | Cation and anion exchange-purified beet thick juice lactic acid | Beet thick juice lactic acid |
|---|---|---|---|---|---|
| Glucose | g/100g | 0 | 27.5 | 17.8 | 27.2 |
| Sucrose | g/100g | 61.2 | 0 | 0 | 0 |
| D-lactic acid | g/L | 0 | 92.6 | 77.6 | 92.6 |
| L-lactic acid | g/L | 0 | 1.74 | 0.51 | 1.74 |
| Optical purity of D-lactic acid | % | - | 96.31 | 98.89 | 96.31 |
| Sodium | mg/L | 158 | 232 | 0 | - |
| Potassium | mg/L | 1300 | ND | ND | - |
| Calcium | mg/L | 26.5 | 23.5 | 17.9 | - |
| Iron | mg/L | 0.529 | 3,513 | 0.028 | - |
| Manganese | mg/L | 0.797 | 0.33 | 0.021 | - |
| Zinc | mg/L | 0.748 | 0.803 | 0 | - |
| Copper | mg/L | 0.019 | 0 | 0 | - |
| Magnesium | mg/L | 0.369 | 19.522 | 0.062 | - |
| Presence or absence of decolorization | - | - | Absent | Present | - |

From Table 2, it can be confirmed that the cation and anion exchange-purified beet thick juice lactic acid exhibits a higher value of the optical purity of D-lactic acid than do the electrodialysis-purified beet thick juice lactic acid and the unpurified beet thick juice lactic acid. Also, it can be confirmed that calcium, iron, manganese, and magnesium remain in the cation and anion exchange-purified beet thick juice lactic acid. Among those, calcium is considered attributable to calcium hydroxide added for salt substitution, similarly to the results of Example 1 and Comparative Example 1. Further, although no decolorization can be confirmed in the electrodialysis-purified beet thick juice lactic acid thus obtained, decolorization can be confirmed in the cation and anion exchange-purified beet thick juice lactic acid.

### <Example 3> Study using L-lactic acid-added sugarcane black treacle

### (1) Preparation of L-lactic acid-added sugarcane black treacle

Because sugarcane black treacle does not contain L-lactic acid, acquisition of sugarcane-derived molasses containing L-lactic acid was attempted. However, the attempt failed due to seasonal reasons. In view of the above, L-lactic acid-added sugarcane black treacle was produced by adding L-lactic acid to sugarcane black treacle, and a study was conducted by using it as pseudo-sugarcane-derived molasses.

Brown sugar syrup (unrefined sugar, treacle, brown sugar; Kokutou Honpo Kakinohana Co., Ltd., containing 65% sucrose) was added to ion exchange water so that the total sucrose content was 100 g/L, to which L-lactic acid (SIGMA) was added so as to have 1.5 g/L to produce L-lactic acid-added sugarcane black treacle.

### (2) Purification of the L-lactic acid-added sugarcane black treacle by the ion exchange resin method

By the same technique as that employed in Example 1 (4), the L-lactic acid-added sugarcane black treacle produced in the present Example 1 (1) was subjected to cation exchange, and then to anion exchange to give positive-and-negative L-lactic acid-added sugarcane black treacle. After carrying out fermentation using the positive-and-negative L-lactic acid-added sugarcane black treacle thus obtained by the same technique as that employed in Example 1 (5), lactic acid (i.e., positive-and-negative L-lactic acid-added sugarcane black treacle lactic acid) was obtained by the same technique as that employed in Example 1 (5). The optical purity of D-lactic acid and the contents of glucose, sucrose, D-lactic acid, and L-lactic acid in the positive-and-negative L-lactic acid-added sugarcane black treacle lactic acid thus obtained were measured by the same technique as that employed in Example 1 (1) and (5). The results of the measurement of the present Example 3 are shown in Table 3.

**[Table 3]**

| | Glucose | Sucrose | Yield from sugar | D-lactic acid | L-lactic acid | Optical purity of D-lactic acid |
|---|---|---|---|---|---|---|
| Unit | g/L | g/L | % | g/L | g/L | % |
| Positive-and-negative L-lactic acid-added sugarcane black treacle lactic acid (g/L) | 19.9 | 35.5 | 45.2 | 22.61 | 0.17 | 98.5 |

### <Example 4> Study on the amount of treacle extruded from an ion exchange resin column

Because the ion exchange resin method is a purification method utilizing chemical adsorption and physical extrusion, the concentration of sucrose will decrease if the collection area is unset. Also, because the ion-adsorbability of resin decreases as the amount of treacle extruded increases, the L-lactic acid-removal capacity decreases. In view of the above, such an extrusion amount of treacle that enables collection of an equal or approximately equal amount of sucrose to the original amount was studied.

A 100 cm-high column having a diameter of 5 cm was filled with 500 mL of anion (strong base ion) exchange resin (Amberlite; Organo Corporation) to form an anion exchange resin column, through which 7 L of an aqueous solution of HB treacle was allowed to pass by the same technique as that employed in Example 1 (2). Subsequently, the anion exchange-purified HB treacle extruded was collected and the amount of HB treacle extruded and the contents of glucose, sucrose, and L-lactic acid in the collected anion exchange-purified HB treacle were measured by the same technique as that employed in Example 1 (1). The results of the measurement, a relationship between the amount of HB treacle extruded and the sucrose content, and the decoloring effect of the ion exchange resin method observed in 1000 to 1800 mL of extruded HB treacle are shown in Figures 4, 5, and 6, respectively.

**[Table 4]**

| Extrualon Amount (mL) | Glucose | | | Sucrose | | | L-laotlo acid | | | Original sucrose | Collected sucrose 2 | Collected sucrose 3 |
|---|---|---|---|---|---|---|---|---|---|---|---|---|
| | Sample No.1 | Sample No.2 | Sample No.3 | Sample No.1 | Sample No2 | Sample No.3 | Sample No.1 | Sample No2 | Sample No.3 | | | |
| 0 | - | - | - | - | - | - | - | - | - | | - | - |
| 100 | 0 | 0 | - | 3.39 | 1.43 | - | 0 | 0 | - | | - | - |
| 200 | 0 | 0 | - | 3.25 | 1.43 | - | 0 | 0 | - | | - | - |
| 300 | 0 | 0 | - | 0.29 | 1.43 | - | 0 | 0 | - | | - | - |
| 400 | 0 | 0 | - | 0.29 | 1.43 | - | 0 | 0 | - | | - | - |
| 500 | 0 | 0 | - | 0.29 | 1.43 | - | 0 | 0 | - | | - | - |
| 600 | 0 | 0 | - | 2.5 | 1.43 | - | 0 | 0 | - | | - | - |
| 700 | 0 | 0 | - | 0.29 | 1.43 | - | 0 | 0 | - | | - | - |
| 800 | 0 | 0 | - | 0.29 | 1.43 | - | 0 | 0 | - | | - | - |
| 900 | 0 | 0 | 0 | 0.29 | 42.99 | 80,08 | 0 | 0 | 0 | | | 183.5 |
| 1000 | 0 | 0 | 3.94 | 43.88 | 94.47 | 204.78 | 0 | 0 | 0 | | | |
| 1100 | 0.25 | 0.23 | 9.9 | 204.56 | 144.02 | 211.11 1 | 0 | 0 | 0 | | | |
| 1200 | 1.22 | 1.23 | 15.07 | 232.55 | 194.76 | 203.68 | 0 | 0 | 0 | 183.9 | | |
| 1300 | 2.81 | 3.28 | 18.18 | 244.67 | 224.68 | 193 | 0 | 0 | 0 | | | |
| 1400 | 6.01 | 8.13 | 20.4 | 237.53 | 229.85 | 180.73 | 0 | 0 | 0 | | | |
| 1500 | 7.49 | 7.91 | 21.54 | 230.89 | 228.04 | 156.7 | 0 | 0 | 0 | | | |
| 1600 | 10.11 | 8.91 | 17.59 | 232.42 | 224.57 | 77.8 | 0 | 0 | 0 | | | |
| 1700 | 9.54 | 8.01 | 7.22 | 149.41 | 223.38 | 34.4 | 0 | 0 | 1.25 | | 94.45 | 20.86 |
| 1800 | 0.25 | 4.02 | 2.34 | 89.7 | 77.51 | 14.62 | 0 | 0 | 0 | | | |
| 2000 | | | 0.87 | | | 7.89 | 0 | 1.155 | 0 | | | |
| 2100 | | | 0.3 | | | 4.56 | 0 | 0 | 0 | | | |
| 2200 | | | 0.08 | | | 2.79 | 0 | 0 | 0 | | | |
| 2300 | | | 0 | | | 0 | 0 | 0 | 0.935 | | | |
| 2400 | | | 0 | | | 0 | 0 | 0 | 0 | | | |
| 2500 | | | 0 | | | 0 | 0 | 0 | 0 | | | |

From Table 4 and Figure 5, it can be confirmed that when the amount of HB treacle extruded is 900 to 2000 mL, an equal or approximately equal amount of sucrose to the original amount can be obtained from the anion exchange-purified HB treacle collected, and further, the L-lactic acid content is 0.0 g/L. That is, it is shown that when the amount of treacle extruded : the amount of anion exchange resin = 1.8 : 1 to 4 : 1 (volume ratio), an equal or approximately equal amount of sucrose to the original amount can be obtained, while L-lactic acid can be surely removed. Also, from Figure 6, it can be confirmed that when the amount of HB treacle extruded exceeds 1600 mL, that is, when it exceeds 3.2 parts by volume per part by volume of anion exchange resin, the decoloring effect of the anion exchange resin column decreases.

### <Example 5> Study on stabilization of culture in the fermentation using cation and anion exchange-purified HB treacle and improvement of the optical purity of D-lactic acid in the resulting lactic acid

From Example 1 (3), it was confirmed that glucose, L-lactic acid, cations, and heavy metals could be removed and lactic acid containing D-lactic acid of high optical purity could be produced by allowing treacle having flowed through a cation exchange resin column where it contacted a cation exchange resin to flow through an anion exchange resin column to allow it to contact an anion exchange resin, or by allowing treacle having flowed through an anion exchange resin column where it contacted an anion exchange resin to flow through an anion exchange resin column to allow it to contact an anion exchange resin. However, there is a tendency that the amount of the resulting D-lactic acid varies depending on the HB treacle used. In view of the above, the effect of potassium dihydrogen phosphate (KH₂PO₄) and magnesium sulfate (MgSO₄) was studied by adding these compounds to cation and anion exchange-purified HB treacle and carrying out culturing and fermentation.

Except for subjecting 350 mL of the cation and anion exchange-purified HB treacle prepared by the same technique as that employed in Example 1 (4), and the above HB treacle to which 0.2 wt/v% of KH₂PO₄ and 0.01 wt/v% of MgSO₄ were added to shaking culture in 500 mL culture flasks containing 350 mL of media at 100 to 150 rpm/minute with a charging stirrer, by the same technique as that employed in Example 1 (5), media were prepared and fermentation was carried out. The resulting cultures were concentration to give lactic acid (i.e., cation and anion exchange-purified HB treacle lactic acid and cation and anion exchange-purified HB treacle addition lactic acid). The optical purities of D-lactic acid in the cation and anion exchange-purified HB treacle lactic acid and the cation and anion exchange-purified HB treacle addition lactic acid per culture time point and the contents of glucose, sucrose, D-lactic acid, and L-lactic acid thus obtained per culture time point were measured by the same technique as that employed in Example 1 (1) and (5). The results of the measurement are shown in Table 5.

**[Table 5]**

| | Glucose | | | Sucrose | | | D-lactic acid | | | L-lactic acid | | |
|---|---|---|---|---|---|---|---|---|---|---|---|---|
| Culture time (hours) | 0 | 24 | 48 | 0 | 24 | 48 | 0 | 24 | 48 | 0 | 24 | 48 |
| Cation and anion exchange-purified HB treacle lactic acid (g/L) | 13.54 | 19.27 | 42.21 | 100 | 30.88 | 0 | 0 | 54.90 | 51.26 | 0 | 0.09 | 0.12 |
| Cation and anion exchange-purified HB treacle addition lactic acidCation and anion exchange-purified HB treacle addition lactic acid | 13.54 | 18.68 | 29.82 | 100 | 34.85 | 0 | 0 | 50.54 | 73.48 | 0 | 0.12 | 0.11 |

From Table 5, it can be confirmed that the contents of glucose in the cation and anion exchange-purified HB treacle lactic acid and the cation and anion exchange-purified HB treacle addition lactic acid were 42.21 g/L and 29.82 g/L, respectively. From this, it is shown that the amount of remaining glucose can be reduced and the yield from sugar can be increased by preparing a medium with addition of KH₂P0₄ and MgSO₄. Further, it can be confirmed that while the contents of L-lactic acid in the cation and anion exchange-purified HB treacle lactic acid and the cation and anion exchange-purified HB treacle addition lactic acid were almost equal with the values being 0.12 g/L and 0.11 g/L, respectively, the contents of D-lactic acid in the cation and anion exchange-purified HB treacle lactic acid and the cation and anion exchange-purified HB treacle addition lactic acid were different with the values being 51.2 g/L and 73.4 g/L, respectively, and the yield of D-lactic acid from sugar can be increased by 43.4% or more.

### <Example 6> Study of a case in which an ion exchange resin column is heated

Using HB treacle, a case in which an ion exchange resin column is heated was studied. Except for heating the ion exchange resin column to 80°C, cation and anion exchange-purified HB treacle was obtained by the same technique as that employed in Example 1 (4). After carrying out fermentation using the cation and anion exchange-purified HB treacle thus obtained by the same technique as that employed in Example 1 (5), lactic acid (i.e., cation and anion exchange-purified HB treacle lactic acid) was obtained by the same technique as that employed in Example 1 (5). The optical purity of D-lactic acid and the yield of D-lactic acid from sugar in the cation and anion exchange-purified HB treacle lactic acid thus obtained were measured by the same technique as that employed in Example 1 (1) and (5). As a result, the optical purity of D-lactic acid was increased to 99.4% e.e. and the yield from sugar was increased to 64.1%.

### <Example 7> Study on scale-up

Based on the aforementioned Examples 1 to 4, the original culture amount was scaled up from 70% of a 10 L jar fermenter (7.0 L) to 65% of a 90 L jar fermenter (58.5 L) in order to mass-produce lactic acid containing D-lactic acid of high optical purity. Except for preparing 58.5 L of cation and anion exchange-purified HB treacle by the same technique as that employed in Example 1 (4) and performing shaking culture at 45°C and 60 rpm/minute, media were prepared and fermentation was carried out, followed by concentration to give lactic acid (i.e., cation and anion exchange-purified HB treacle lactic acid) by the same technique as that employed in Example 1 (5). A relationship among the culture time before and after scale-up, the pH value, and the amount of sucrose consumed is shown in Figure 7.

From Figure 7, it can be confirmed that the amount of sucrose after scaling up to 90 L is consumed in a culture time of approximately 500 minutes, and pH is also stabilized, as in the case of the 10 L-scale. Also, as a result of measuring the optical purity of D-lactic acid and the contents of glucose, sucrose, D-lactic acid, and L-lactic acid after scaling up to 90 L by the same technique as that employed in Example 1 (1) and (5), the amounts of D-lactic acid and L-lactic acid were found to be 276.0 g/L and 0.8 g/L, respectively, and D-lactic acid having an optical purity of 99.4% e.e. was obtained, and the collection rate of D-lactic acid was 43%.

Compared to an existing method, which includes allowing microorganisms to utilize treacle and culturing the microorganisms, lactic acid containing D-lactic acid of higher optical purity can be efficiently produced, and further, the yield of D-lactic acid from sugar can be increased according to the present Examples as shown above.

Also, the method for producing D-lactic acid and the method for increasing the optical purity of D-lactic acid in lactic acid according to the present invention are not limited to the aforementioned embodiments but can be appropriately modified.

## Claims

1. A method for producing D-lactic acid by allowing microorganisms to utilize treacle and culturing the microorganisms, the method comprising the step of purifying the treacle using an ion exchange resin method.

2. The method for producing D-lactic acid according to claim 1, wherein the microorganisms are lactic acid bacteria.

3. The method for producing D-lactic acid according to claim 1 or 2, wherein the ion exchange resin method uses two or more ion exchange resins and comprises the step of contacting treacle having been contacted with a cation exchange resin or an anion exchange resin with an anion exchange resin.

4. The method for producing D-lactic acid according to any of claims 1 to 3, wherein the ion exchange resin method is a column method using two or more ion exchange resin columns and comprising allowing treacle having flowed through a cation exchange resin column or an anion exchange resin column to flow through an anion exchange resin column.

5. The method for producing D-lactic acid according to claim 4, wherein, in the column method, an amount of treacle extruded from the anion exchange resin column used first is 1.8 to 4 parts by volume per part by volume of the anion exchange resin.

6. The method for producing D-lactic acid according to any of claims 1 to 5, wherein the ion exchange resin method or the column method uses a heated ion exchange resin or ion exchange resin column.

7. The method for producing D-lactic acid according to any of claims 1 to 6, wherein the treacle has one or two or more ions selected from the group consisting of potassium ions, magnesium ions, phosphate ions, hydrogen phosphate ions, dihydrogen phosphate ions, and sulfate ions added thereto.

8. A method for increasing an optical purity of D-lactic acid in lactic acid, comprising the step of purifying treacle using an ion exchange resin method.

9. The method for increasing an optical purity of D-lactic acid in lactic acid according to claim 8, wherein the microorganisms are lactic acid bacteria.

10. The method for increasing an optical purity of D-lactic acid in lactic acid according to claim 8 or 9, wherein the ion exchange resin method uses two or more ion exchange resins and comprises the step of contacting treacle having been contacted with a cation exchange resin or an anion exchange resin with an anion exchange resin.

11. The method for increasing an optical purity of D-lactic acid in lactic acid according to any of claims 8 to 10, wherein the ion exchange resin method is a column method using two or more ion exchange resin columns and comprising allowing treacle having flowed through a cation exchange resin column or an anion exchange resin column to flow through an anion exchange resin column.

12. The method for increasing an optical purity of D-lactic acid in lactic acid according to claim 11, wherein, in the column method, an amount of treacle extruded from the anion exchange resin column used first is 1.8 to 4 parts by volume per part by volume of anion exchange resin.

13. The method for increasing an optical purity of D-lactic acid in lactic acid according to any of claims 8 to 12, wherein the ion exchange resin method or the column method uses a heated ion exchange resin or ion exchange resin column.

14. A method for increasing a yield of D-lactic acid from sugar, comprising the step of allowing microorganisms to utilize treacle having one or two or more ions selected from the group consisting of potassium ions, magnesium ions, phosphate ions, hydrogen phosphate ions, dihydrogen phosphate ions, and sulfate ions added thereto and culturing the microorganisms.

15. Lactic acid comprising D-lactic acid, an optical purity of the D-lactic acid or a yield of the D-lactic acid from sugar being increased using the method according to any of claims 8 to 14.
